# EUROPÄISCHE PATENTANMELDUNG

(11) **EP 4 094 806 A1**
(43) Veröffentlichungstag der Anmeldung: **30.11.2022**
(21) Anmeldenummer: 22174827.0
(22) Anmeldetag: 23.05.2022
(51) Int. Cl.: A62B 9/06, A61M 16/06, A62B 18/02

(54) **PATIENTENSCHNITTSTELLE MIT EINER DICHTEINRICHTUNG AUFWEISEND EINE STÜTZMEMBRAN UND EINE DICHTMEMBRAN**

(30) Priorität: 28.05.2021 DE 102021002769
(71) Anmelder: Löwenstein Medical Technology S.A., 2557 Luxembourg (LU)
(72) Erfinder: Eifler, Martin, 25348 Glückstadt (DE); Gottschewski, Anja, 20126 Hamburg (DE)
(74) Vertreter: Marx, Thomas

(57) **Zusammenfassung**

Die Erfindung betrifft eine Patientenschnittstelle mit einer Dichteinrichtung, wobei die Dichteinrichtung eine Stützmembran und eine Dichtmembran aufweist, wobei die Dichtmembran zumindest abschnittsweise zur Anlage an der Haut eines Patienten ausgebildet ist dadurch gekennzeichnet, dass die Stützmembran zumindest abschnittsweise benachbart zur Dichtmembran verläuft und wobei der Abstand zwischen der Stützmembran und der Dichtmembran zumindest abschnittsweise derart ausgebildet ist, dass ein Zwischenraum gebildet wird.

## Beschreibung

Die Erfindung betrifft eine Patientenschnittstelle mit einer Dichteinrichtung, wobei die Dichteinrichtung eine Stützmembran und eine Dichtmembran aufweist, wobei die Dichtmembran zumindest abschnittsweise zur Anlage an der Haut eines Patienten ausgebildet ist dadurch gekennzeichnet, dass die Stützmembran zumindest abschnittsweise benachbart zur Dichtmembran verläuft und wobei der Abstand zwischen der Stützmembran und der Dichtmembran zumindest abschnittsweise derart ausgebildet ist, dass ein Zwischenraum gebildet wird.

Der Abstand zwischen der Stützmembran und der Dichtmembran liegt zumindest abschnittsweise bei zumindest 1 bis 5 mm.

Die Patientenschnittstelle ist auch dadurch gekennzeichnet, dass die Dichteinrichtung ein Randprofil mit einer Basis aufweist, wobei sich die Dichtmembran und die Stützmembran von der gemeinsamen Basis ausgehend und beabstandet zueinander erstrecken.

Die Patientenschnittstelle ist auch dadurch gekennzeichnet, dass die Dichteinrichtung ein näherungsweise U-förmiges Randprofil aufweist, welches einen Befestigungsbereich zur formschlüssigen Verbindung der Dichteinrichtung mit einem Maskenkörper bildet.

Die Patientenschnittstelle ist auch dadurch gekennzeichnet, dass der Befestigungsbereich durch eine umlaufende, offene Nut gebildet ist.

Die Patientenschnittstelle ist auch dadurch gekennzeichnet, dass das Randprofil zwei Profilwände, aufweist die die Nut bilden.

Die Patientenschnittstelle ist auch dadurch gekennzeichnet, dass die Nut trichterförmig gestaltet ist.

Die Patientenschnittstelle ist auch dadurch gekennzeichnet, dass die Nut dadurch trichterförmig gestaltet ist, dass zumindest eine der Profilwände, einen abgeschrägten Wandabschnitt aufweist, der mit seiner Schräge (abgeschrägter Wandabschnitt) von der gegenüberliegenden Profilwand zurückspringt.

Die Patientenschnittstelle ist auch dadurch gekennzeichnet, dass die Profilwand eine Nase aufweist.

Die Patientenschnittstelle ist auch dadurch gekennzeichnet, dass die Profilwand einen abgeschrägten Wandabschnitt aufweist, der zu der Basis des U-förmigen Randprofils weist.

Die Patientenschnittstelle ist auch dadurch gekennzeichnet, dass der abgeschrägte Wandabschnitt einen Hinterschnitt für die Aufweitung am Gegenprofil des Maskenkörpers aufweist, wobei das Randprofil so ein Gegenprofil eines Maskenkörpers aufnehmen kann.

Die Patientenschnittstelle ist auch dadurch gekennzeichnet, dass die Membranen, von dem Randprofil ausgehen und in eine von der Nut abgewandte Richtung weisen.

Die Patientenschnittstelle ist auch dadurch gekennzeichnet, dass die Stützmembran kürzer ausgebildet ist als die Dichtmembran, wobei die Stützmembran zumindest abschnittsweise von der Dichtmembran überdeckt wird.

Insofern ist sichergestellt, dass das Patientengesicht üblicherweise nur mir der Dichtmembran in Kontakt tritt.

Die Patientenschnittstelle ist auch dadurch gekennzeichnet, dass von dem Randprofil überall oder umlaufend eine Dichtmembran entspringt.

Die Patientenschnittstelle ist auch dadurch gekennzeichnet, dass von dem Randprofil bereichsweise keine Stützmembran entspringt. Die Stützmembran also nicht durchgängig ausgebildet ist

Der Nasenrückenbereich, also der Bereich der Dichteinrichtung, der zur Anlage an den Nasenrücken ausgebildet ist, wird von der Stützmembran ausgespart.

Der Kinnbereich, also der Bereich der Dichteinrichtung, der zur Anlage an dem Kinn oder der Oberlippe oder der Unterlippe ausgebildet ist, wird von der Stützmembran ausgespart.

Die Patientenschnittstelle ist auch dadurch gekennzeichnet, dass ein Kontaktbereich zur Haut eines Patienten durch die Dichtmembran gebildet wird, die im Kontaktbereich als Dichtlippe ausgeformt ist.

Die Patientenschnittstelle ist auch dadurch gekennzeichnet, dass die Dichtmembran sich von der Basis des Randprofils ausgehend strahlförmig erstreckt und dann gerundet in eine Richtung (zu einem Innenraum, in den Nase und oder Mund teilweise eingeführt werden) abknickt, wobei die Dichtmembran zumindest abschnittsweise einen rechten Winkel zu sich selbst bildet.

Die Patientenschnittstelle ist auch dadurch gekennzeichnet, dass die Stützmembran zumindest abschnittsweise im Querschnitt dicker als die Dichtmembran ist.

Die Patientenschnittstelle ist auch dadurch gekennzeichnet, dass die Membranen,, beabstandet voneinander in der Basis entspringen, wobei die Dichtmembran als Verlängerung der Wand ausgeführt ist, wobei die Wandstärke der Dichtmembran geringer ist als die Wandstärke der Wand .

Die Patientenschnittstelle ist auch dadurch gekennzeichnet, dass die Wandstärke der Dichtmembran im weiteren Verlauf eine gleichbleibende oder nur wenig abnehmende Wandstärke aufweist.

Die Patientenschnittstelle ist auch dadurch gekennzeichnet, dass die Stützmembran, im Wesentlichen mit einem Versatz, als Verlängerung der Wand ausgeführt ist.

Die Patientenschnittstelle ist auch dadurch gekennzeichnet, dass die Wandstärke der Stützmembran im Wesentlichen der Wandstärke der Wand entspricht, wobei die Wandstärke der Stützmembran im weiteren Verlauf (hin zu der Dichtlippe ) eine abnehmende Wandstärke aufweist.

Die Patientenschnittstelle ist auch dadurch gekennzeichnet, dass die Membranen, einen elastischen mantelförmigen Wandabschnitt ausbilden und in Dichtlippen, enden.

Die Patientenschnittstelle ist auch dadurch gekennzeichnet, dass die Wandstärke der Dichtmembran im Verlauf hin zu der Dichtlippe eine gleichbleibende oder nur wenig abnehmende Wandstärke aufweist, wobei die Wandstärke der Dichtmembran im Bereich 0,15 mm bis 0,6 mm ist.

Bevorzugt ist die Wandstärke im Bereich 0,25 bis 0,45 mm, besonders bevorzugt im Bereich 0,3 mm bis 0,4 mm.

Die Patientenschnittstelle ist auch dadurch gekennzeichnet, dass die Wandstärke der Stützmembran an der Stelle, wo die Stützmembran der Basis entspringt, im Bereich 2,9 mm bis 1,4 mm liegt.

Bevorzugt ist die Wandstärke im Bereich 2,1 bis 1,6 mm, besonders bevorzugt im Bereich 2,0 mm bis 1,7 mm, ganz besonders bevorzugt bei 1,8 mm.

Die Patientenschnittstelle ist auch dadurch gekennzeichnet, dass die Wandstärke der Stützmembran im Bereich der Dichtlippe im Bereich zwischen 0,7 mm bis 0,25 mm liegt.

Bevorzugt ist die Wandstärke im Bereich 0,65 bis 0,4 mm, besonders bevorzugt im Bereich 0,6 mm bis 0,45 mm, ganz besonders bevorzugt bei 0,5 mm.

Die Patientenschnittstelle ist auch dadurch gekennzeichnet, dass die Wandstärke der Dichtlippen, im Wesentlichen identisch ist.

Die Patientenschnittstelle ist auch dadurch gekennzeichnet, dass der Abstand zwischen Dichtmembran und der Stützmembran an der Basis bei 0,5 bis 3,5 mm liegt, wobei die Membranen von dort aus mit einem Winkel von größer als 1°, bevorzugt von 1,3 bis 4°, besonders bevorzugt von 3° auseinander laufen.

Der leichte Winkel führt dazu, dass die Stützmembran und die Dichtmembran nicht aneinanderkleben oder zueinander laufen, sondern divergieren. Dadurch wird eine Durchströmung des von den Membranen, zumindest teilweise umgebenen Zwischenraums mit Atemgas ermöglicht. Die Durchströmung des von den Membranen, zumindest teilweise umgebenen Zwischenraums mit Atemgas hat zumindest zwei vorteilhafte Wirkungen. Erstens kann der anliegende Therapiedruck die Dichtmembran zumindest leicht nach außen aufblähen oder gegen das Gesicht des Patienten drücken. Zweitens wird durch die Durchströmung des Zwischenraums eine Ansammlung kondensierender Feuchtigkeit aus der Atemluft oder dem Atemgas zumindest abgemildert oder teilweise verhindert.

Die Patientenschnittstelle ist auch dadurch gekennzeichnet, dass der Abstand zwischen den Membranen und ist über den Bereich der gemeinsamen Erstreckung im Wesentlichen bei zumindest 1 bis 4 mm, bevorzugt bei 1,4 bis 3 mm, besonders bevorzugt bei 1,5 bis 1,8 mm oder bei 2 mm liegt.

Die Patientenschnittstelle ist auch dadurch gekennzeichnet, dass die Dichteinrichtung aus Silikon oder einem anderen elastischen Material hergestellt ist, wobei die Dichteinrichtung eine Stützmembran und eine Dichtmembran umfasst, wobei die Dichtmembran einen elastischen, mantelartigen Wandabschnitt bildet der die Dichteinrichtung außen begrenzt und die Stützmembran zumindest teilweise überdeckt oder überlappt, wobei die Elastizität des elastischen mantelartigen Wandabschnitts eine Bewegung der Membranen, relativ zu dem durch das Randprofil gebildeten Befestigungsbereich der Dichteinrichtung ermöglicht, insbesondere eine Verschiebung senkrecht zur Längsachse und/oder ein Kippen oder Schwenken um Achsen senkrecht zur Längsachse.

Die Patientenschnittstelle ist auch dadurch gekennzeichnet, dass der Maskenkörper und die Dichteinrichtung zusammen einen Innenraum oder Atemgasraum bilden, in welchen das Gesicht des Anwenders/Patienten bei der Anwendung der Maske auf dem Gesicht zumindest teilweise hereinragt, wobei der Innenraum der Patientenschnittstelle über ein Ausatemsystem mit dem Außenbereich der Patientenschnittstelle gasleitend verbunden ist und wobei über einen Schlauchanschluss die Patientenschnittstelle über einen Beatmungsschlauch mit einem Beatmungsgerät gasleitend verbunden ist, wobei die Dichteinrichtung einen Kontaktbereich aufweist, der zur dichtenden Anlage an das Gesicht eines Patienten ausgebildet ist, der im Wesentlichen durch die Dichtlippe gebildet ist.

Die Patientenschnittstelle ist auch dadurch gekennzeichnet, dass der Kontaktbereich einen Profilverlauf aufweist, der so ausgebildet ist, dass der Kontaktbereich zur Anlage auf einem Nasenrücken und zur Anlage entlang einer Nasolabialfalte und entlang einer Oberlippe oder Unterlippe oder eines Kinnbereiches eines Patienten ausgebildet ist.

Die Stützmembran ist üblicherweise nicht oder zumindest weniger stark ausgeprägt im Nasenrücken- und im Oberlippen- oder Unterlippen- oder Kinn-bereich.

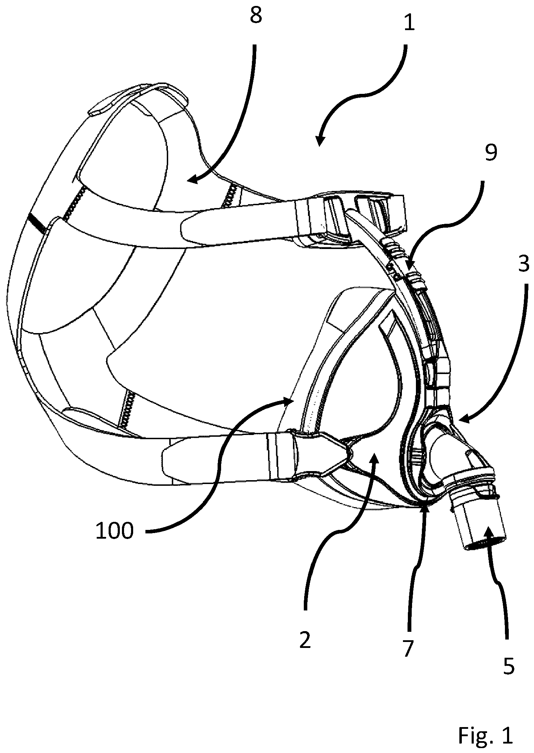

Figur 1 zeigt eine beispielhafte Ausführungsform der Patientenschnittstelle 1 als Maske mit einer beispielhaften Ausführungsform des Ausatemsystems 3. Die Patientenschnittstelle 1 umfasst beispielhaft einen Maskenkörper 2, an welchen eine Stirnstütze 9 und eine Kopfbänderung 8 angeordnet sind. Der Verschluss 7 bildet beispielhaft zusammen mit Teilen des Maskenkörpers 2 das Ausatemsystem 3. Der Verschluss 7 ist beispielhaft zudem so eingerichtet, dass ein Gelenkkopf eines Kugelgelenks 6 aufgenommen werden kann. Über das Kugelgelenk 6 ist ein Schlauchanschluss 5 mit der Patientenschnittstelle 1 verbunden. Mit dem Maskenkörper 2 ist ein Dichteinrichtung 100 verbunden, welches dazu ausgebildet ist mit einer Fläche auf dem Gesicht eines Anwenders/Patienten aufzuliegen. Zudem umfasst die Patientenschnittstelle eine Dichteinrichtung 100, welche dazu ausgebildet ist zumindest Teile des Gesichts, wie zum Beispiel Nase und/oder Mund, aufzunehmen. Der Maskenkörper 2 und die Dichteinrichtung 100 umschließen zusammen einen Innenraum (im Folgenden vereinfacht Innenraum der Patientenschnittstelle 1), in welchen das Gesicht des Anwenders/Patienten bei der Anwendung der Maske auf dem Gesicht zumindest teilweise hereinragt. Die Dichteinrichtung 100 ist beispielsweise über ein Profil 200 mit dem Maskenkörper 2 verbunden. In manchen Ausführungsformen ist die Dichteinrichtung 100 auch Teil des Maskenkörpers. Der Innenraum der Patientenschnittstelle 1 ist über das Ausatemsystem 3 mit dem Außenbereich der Patientenschnittstelle 1 gasleitend verbunden. Über den Schlauchanschluss 5 kann die Patientenschnittstelle 1 beispielsweise über einen Beatmungsschlauch mit einem Beatmungsgerät gasleitend verbunden werden. Über den Schlauchanschluss 5 kann so zum Beispiel Atemgas in und/oder aus dem Innenraum der Patientenschnittstelle 1 und dem Anwender/Patienten geleitet bzw. gefördert werden. Beispielsweise ist die Patientenschnittstelle 1 als Full-Face Maske ausgeführt, sodass von der Dichteinrichtung 100 zumindest Nase und Mund des Anwenders umschlossen werden. Die erfinderische Dichteinrichtung 100 kann aber auch in anderen Patientenschnittstellen 1, wie beispielsweise Nasal-Masken, Trachealkanülen/Trachealtuben und/oder Nasenbrillen Anwendung finden.

Beispielsweise wird durch den Schlauchanschluss 5 und das Kugelgelenk 6 Atemgas von einem Beatmungsgerät in den Innenraum 400 der Patientenschnittstelle 1 gefördert um den Anwender/Patienten bei der Atmung zu unterstützen. Die Förderung des Atemgases wird dazu beispielsweise über den Druck oder den Fluss kontrolliert. Durch das optionale Ausatemsystem 3 kann gewollt ständig Atemgas entweichen, wodurch auch ausgeatmetes Atemgas des Patienten/Anwenders aus dem Innenraum der Patientenschnittstelle 1 gefördert bzw. ausgewaschen wird. So wird eine Anreicherung von CO2 in dem Atemgas bzw. in dem Innenraum der Patientenschnittstelle 1 verhindert und der Patienten/Anwender kann frisches Atemgas einatmen.

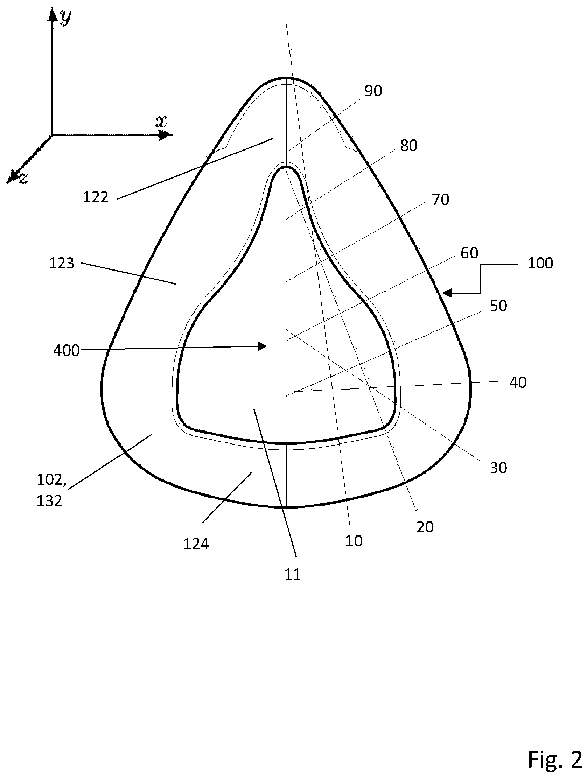

Figur 2 zeigt eine schematische Darstellung der Dichteinrichtung 100 mit unterschiedlichen Schnittebenen 10, 20, 30 ...90. Die Dichteinrichtung 100 ist in einer Draufsicht auf den Kontaktbereich 132, der zur Anlage an das Gesicht eines nicht dargestellten Patienten ausgebildet ist, gezeigt, die im Wesentlichen durch die Dichtlippe 102 gebildet ist.

Die Dichteinrichtung hat eine vertikale Erstreckung in einer y-Richtung und eine horizontale Erstreckung in einer x-Richtung. Die maximale vertikale Erstreckung ist größer als die maximale horizontale Erstreckung. Die maximale vertikale Erstreckung ist 5 -15 cm und die maximale horizontale Erstreckung ist 4-14 cm. Der Kontaktbereich 132 mit der Dichtlippe 102 umgrenzt den Atemgasraum (innen) 400, in den Mund und/oder Nase eines nicht gezeigten Patienten eingeführt werden. Der Atemgasraum 400 wird im Zusammenspiel von Maskenkörper 2 und Dichteinrichtung 100 gebildet.

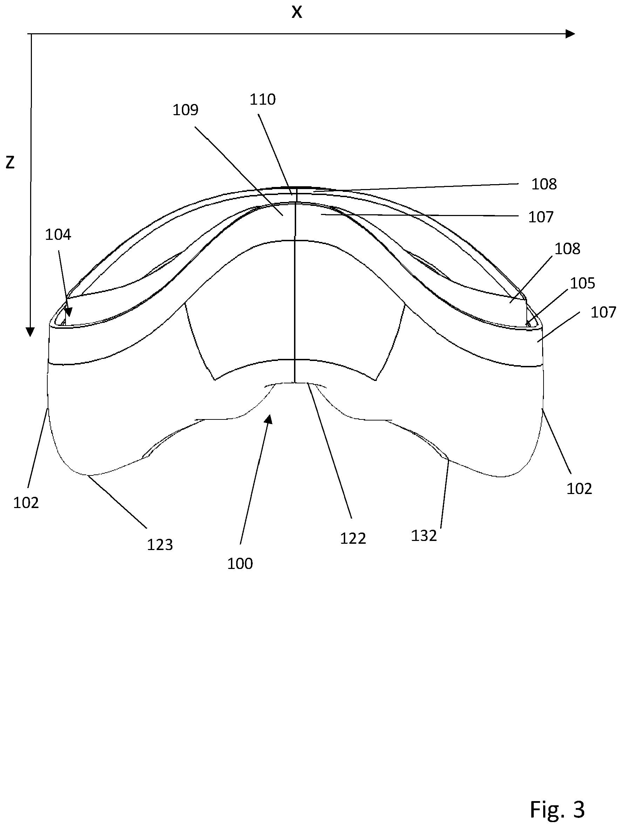

Figur 3 zeigt eine schematische Darstellung der Dichteinrichtung 100 in einer perspektivischen Ansicht. Die Dichteinrichtung 100 ist in einer Draufsicht von oben gezeigt. In einer x-Richtung ist die maximale horizontale Erstreckung erkennbar die im Wesentlichen durch die Außenkante der Dichtlippe 102 begrenzt ist.

In einer z-Richtung ist die maximale Tiefe erkennbar die zum einen durch den Kontaktbereich 132 der Dichtlippe 102, der zur Anlage an das Gesicht eines nicht dargestellten Patienten ausgebildet ist, begrenzt ist. Eine weitere Begrenzung sind die Wände 107 und 108 des Randprofils 104, die eine Nut 105 zwischen sich ausbilden und zur Verbindung mit einem nicht dargestellten Maskenkörper ausgebildet sind.

Die Dichteinrichtung 100 hat eine vertikale Erstreckung in einer z-Richtung und eine horizontale Erstreckung in einer x-Richtung. Die maximale vertikale 11 Erstreckung ist geringer als die maximale horizontale Erstreckung. Die maximale vertikale Erstreckung ist 5 - 15 cm und die maximale horizontale Erstreckung ist 4 - 14 cm.

Die Dichteinrichtung 100 weist einen Höhenverlauf auf. Erkennbar ist dieser Höhenverlauf beispielsweise an dem Verlauf der Wände 107 und 108 des Randprofils 104. Die Wände 107, 108 sind in dem Bereich der Spitze 109 und der Basis 110 der Dichteinrichtung 100 der nahe an der x-Achse angeordnet. Die Wände 107, 108 sind im Bereich der Außenkante der Dichtlippe 102 entfernt von der x-Achse angeordnet.

Der Kontaktbereich 132 weist einen entsprechenden Verlauf auf. Der Kontaktbereich 132 der zur Anlage auf einem nicht dargestellten Nasenrücken ausgebildet ist - der Nasenrückenbereich 122, ist nahe an der x-Achse angeordnet.

Der Kontaktbereich 132 der zur Anlage auf einem nicht dargestellten Kinn oder Ober- oder Unterlippe ausgebildet ist - der Kinnbereich 124, ist nahe an der x-Achse angeordnet.

Der Kontaktbereich 132 der zur Anlage auf den nicht dargestellten Wangen bzw. der Nasolabialfalte ausgebildet ist - der Nasolabialbereich 123, ist entfernt zu der x-Achse angeordnet.

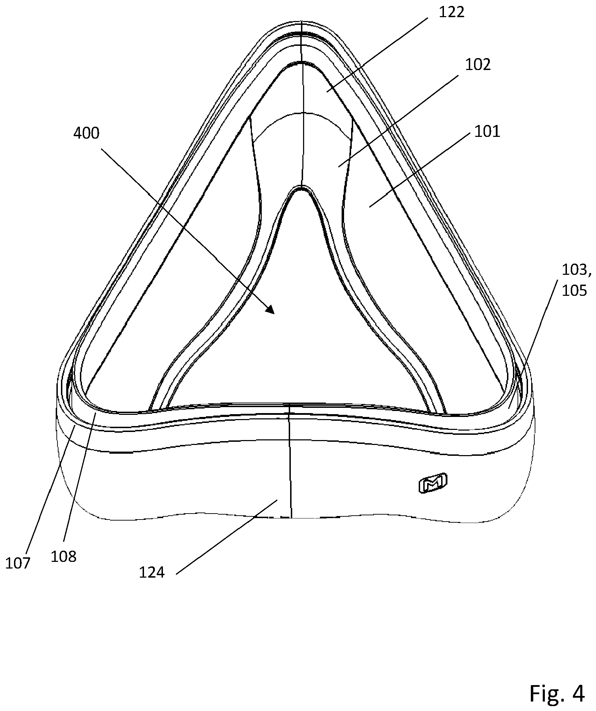

Figur 4 zeigt eine schematische Darstellung der Dichteinrichtung 100 in einer perspektivischen Ansicht auf das Randprofil 103 als Kontaktstelle zum Maskenkörper 2. Die Dichteinrichtung 100 ist in einer Draufsicht von unten gezeigt.

In dieser Ansicht ist die annähernd dreieickförmige Grundform der Dichteinrichtung 100 erkennbar. Die Dichteinrichtung 100 weist ein näherungsweise U-förmiges Randprofil 103 auf, welches einen Befestigungsbereich zur formschlüssigen Verbindung der Dichteinrichtung 100 mit einem Maskenkörper 2 bildet. Das Randprofil 103 wird durch eine umlaufende, offene Nut 105 gebildet. Das Randprofil 103 weist zwei Profilwände 108, 107 auf die die Nut 105 bilden. Die Dichtmembranen 101, 102 gehen von dem Randprofil aus und weisen, in eine von der Nut 105 abgewandte Richtung, zu einem nicht gezeigten Patientengesicht hin. Die Stützmembran 101 ist kürzer ausgebildet und wird zumindest abschnittsweise (hier vollständig) von der Dichtmembran 102 überdeckt. Insofern ist sichergestellt, dass das Patientengesicht üblicherweise nur mir der Dichtmembran 102 in Kontakt tritt. Offensichtlich ist auch, dass die Stützmembran 101 nicht durchgängig ausgebildet ist. Der Nasenrückenbereich 122 und der Kinnbereich 124 werden von der Stützmembran 101 ausgespart.

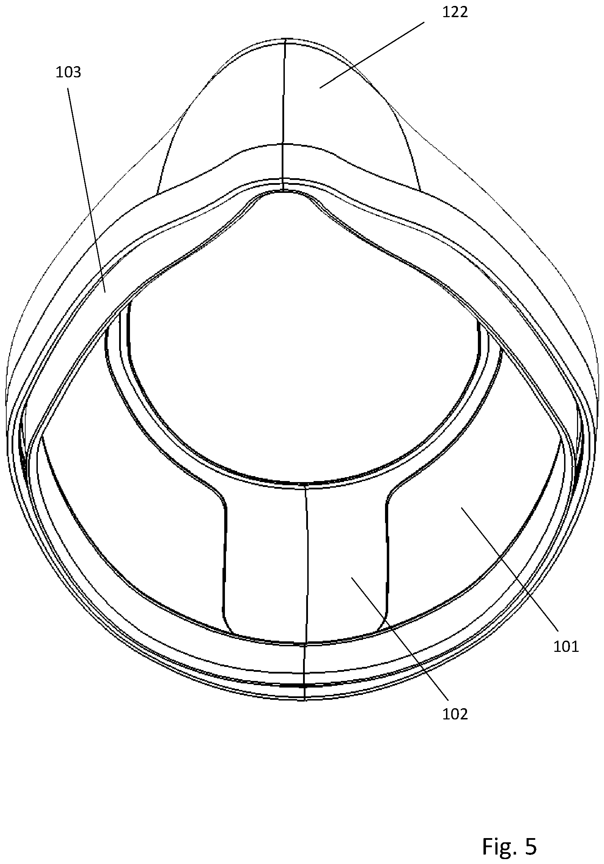

Figur 5 zeigt eine schematische Darstellung der Dichteinrichtung 100 in einer perspektivischen Ansicht auf das Randprofil 103 als Kontaktstelle zum Maskenkörper 2. Die Dichteinrichtung 100 ist in einer Draufsicht von oben gezeigt. Offensichtlich ist, dass die Stützmembran 101 nicht durchgängig ausgebildet ist. Der Kinnbereich 124 wird von der Stützmembran 101 ausgespart. Man erkennt auch, dass die Dichtmembran die Stützmembran überlappt.

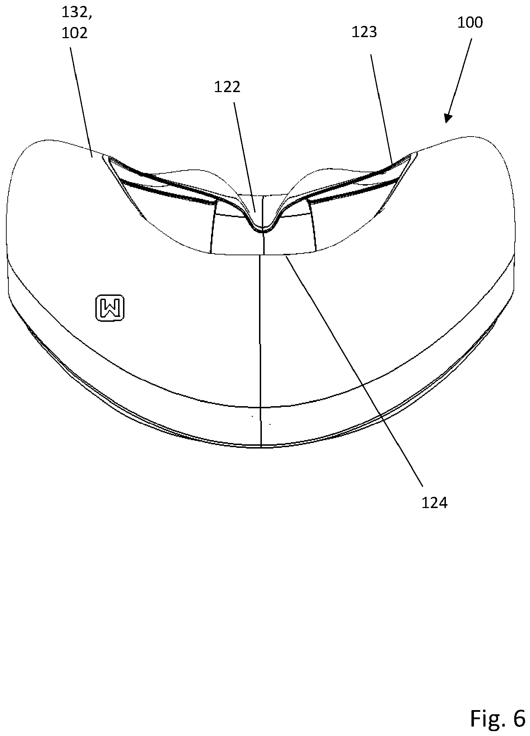

Figur 6 zeigt eine schematische Darstellung der Dichteinrichtung 100 in einer perspektivischen Ansicht auf den Kontaktbereich 132 als Kontaktstelle zum Patienten. Die Dichteinrichtung 100 ist in einer Draufsicht von unten gezeigt. Der Kontaktbereich 132 wird insbesondere durch die Dichtmembran 102 gebildet, die im Kontaktbereich 132 als Dichtlippe 112 ausgeformt ist. Der Nasenrückenbereich 122 und der Kinnbereich werden von der Stützmembran 101 ausgespart. Zudem ist der Höhenverlauf oder das Profil erkennbar. Die Dichteinrichtung 100 weist eine Einbuchtung im Nasenrückenbereich 122 und eine Einbuchtung im Kinnbereich 124 auf. Der seitliche Bereich Nasolabialbereich 123 ist demgegenüber erhöht ausgebildet. Der Bereich, der beidseitig unmittelbar benachbart zu der Einbuchtung im Nasenrückenbereich 122 angeordnet ist, ist ebenfalls erhöht ausgebildet.

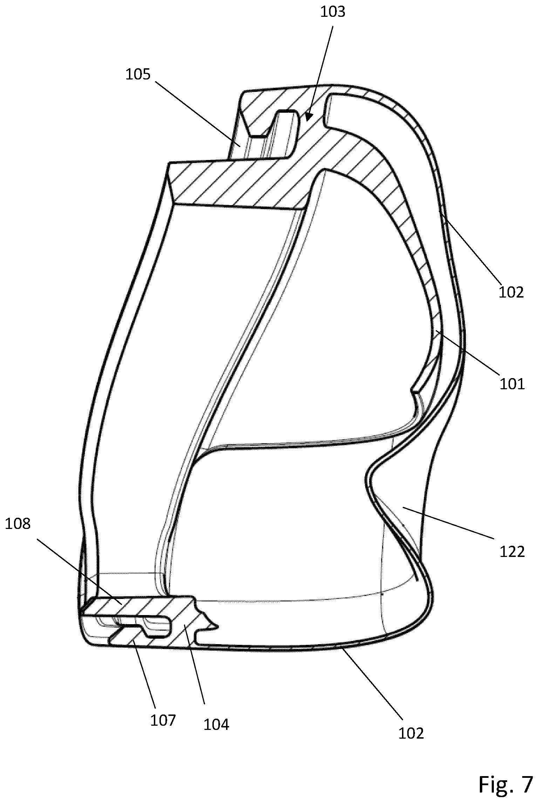

Figur 7 zeigt eine schematische Darstellung eines Schnitts durch eine Dichteinrichtung 100 gemäß Figur 3 an der Position 90, im Nasenrückenbereich 122. Die Dichteinrichtung 100 weist ein näherungsweise U-förmiges Randprofil 103 auf, welches einen Befestigungsbereich zur formschlüssigen Verbindung der Dichteinrichtung 100 mit einem Maskenkörper 2 bildet. Das Randprofil 103 wird durch eine umlaufende, offene Nut 105 gebildet. Das Randprofil 103 weist zwei Profilwände 108, 107 auf die die Nut 105 bilden.

Man erkennt, dass die Stützmembran 101 nicht durchgängig ausgebildet ist. Der Nasenrückenbereich 122 wird von der Stützmembran 101 ausgespart. Die Dichtmembran 102 erstreckt sich von der Basis 104 des Randprofil 103 ausgehend strahlförmig und biegt dann gerundet in eine Richtung, die zum Innenraum 400 weist, ab, wobei die Dichtmembran 102 zumindest abschnittsweise einen rechten Winkel zu sich selbst bildet. Die Stützmembran 101 erstreckt sich ebenfalls von der Basis 104 des Randprofil 103 im Wesentlichen parallel zu der Dichtmembran 102. Die Stützmembran 101 ist dicker als die Dichtmembran 102.

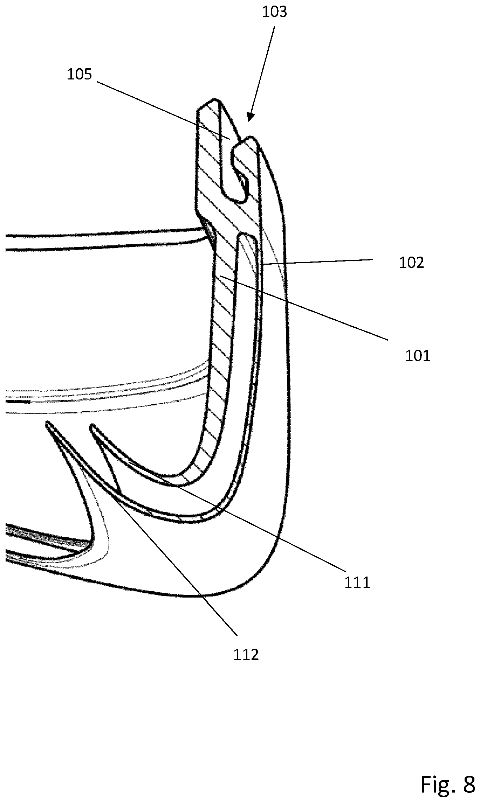

Figur 8 zeigt eine schematische Darstellung eines Schnitts durch eine Dichteinrichtung 100 gemäß Figur 3 an der Position 50, am unteren Ende des Nasolabialbereiches 123. Die Dichteinrichtung 100 weist ein näherungsweise U-förmiges Randprofil 103 auf, welches einen Befestigungsbereich zur formschlüssigen Verbindung der Dichteinrichtung 100 mit einem Maskenkörper 2 bildet. Das Randprofil 103 wird durch eine umlaufende, offene Nut 105 gebildet. Man erkennt, dass die Stützmembran 101 hier durchgängig ausgebildet ist. Der Nasolabialbereich 123 wird von der Stützmembran 101 nicht ausgespart und stützt hier die Maske am Gesicht des Patienten ab. Die Dichtmembran 102 erstreckt sich von der Basis 104 des Randprofil 103 ausgehend strahlförmig und biegt dann gerundet in eine Richtung, die zum Innenraum 400 weist, ab, wobei die Dichtmembran 102 zumindest abschnittsweise einen rechten Winkel zu sich selbst bildet. Die Stützmembran 101 erstreckt sich ebenfalls von der Basis 104 des Randprofil 103 im Wesentlichen parallel zu der Dichtmembran 102. Die Stützmembran 101 ist dicker als die Dichtmembran 102, wobei die Stützmembran 101 einen Dickenverlauf aufweist. Die Stützmembran 101 und die Dichtmembran 102 enden in Dichtlippen 111 bzw. 112. Die Dichtlippen können in einem Querschnitt dieselbe Dicke aufweisen.

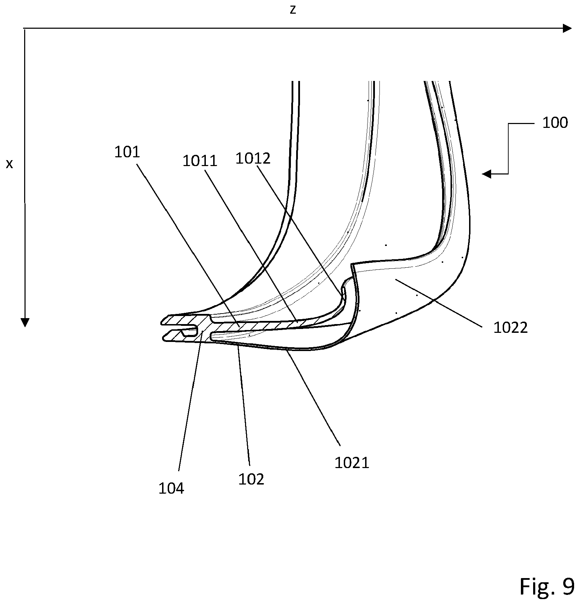

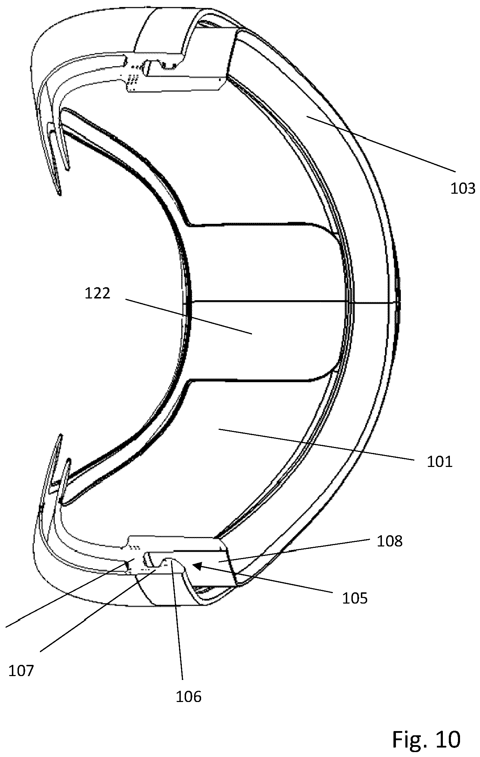

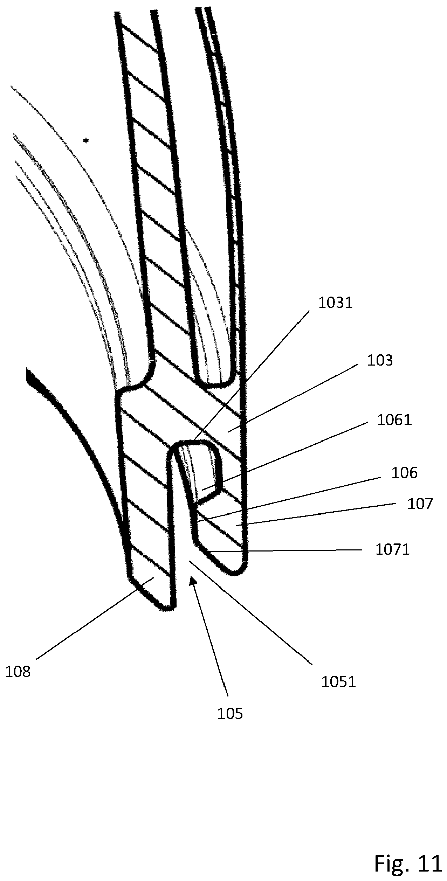

Bei dem in Figur 9 dargestellten Ausführungsbeispiel sind die Dichtmembranen 101, 102 zumindest abschnittsweise gewölbt oder gerundet ausgeführt. Insbesondere sind die Dichtmembran 101, und die Dichtmembran 102 von der Basis 104 ausgehend in einer z-Richtung strahlförmig und dann in eine x-Richtung abknickend oder gerundet ausgebildet. Die Dichtmembran 102 erstreckt sich von der Basis 104 ausgehend in einer z-Richtung strahlförmig und biegt dann in gerundet in eine x-Richtung ab, wobei die Dichtmembran 102 zumindest abschnittsweise einen rechten Winkel zu sich selbst bildet. Die Dichtmembran 102 erstreckt sich von der Basis 104 ausgehend in einer z-Richtung strahlförmig mit einer ersten Ausdehnung 1021 und biegt dann in gerundet in eine x-Richtung mit einer zweiten Ausdehnung 1022 ab, wobei die erste Ausdehnung 1021 länger ist als die zweite Ausdehnung 1022. Dichtmembran 101, wobei die erste Ausdehnung 1021 mehr als 20 Prozent länger ist als die zweite Ausdehnung 1022.

Die Stützmembran 101 erstreckt sich von der Basis 104 ausgehend in einer z-Richtung strahlförmig und biegt dann in gerundet in eine x-Richtung ab, wobei die Stützmembran 101 zumindest abschnittsweise einen rechten Winkel zu sich selbst bildet. Die Stützmembran 101 erstreckt sich von der Basis 104 ausgehend in einer z-Richtung strahlförmig mit einer ersten Ausdehnung 1011 und biegt dann in gerundet in eine x-Richtung mit einer zweiten Ausdehnung 1012 ab, wobei die erste Ausdehnung 1011 länger ist als die zweite Ausdehnung 1012. Dichtmembran 101, wobei die erste Ausdehnung 1011 mehr als 30 Prozent länger ist als die zweite Ausdehnung 1012.

Die Dichtmembran 102 überdeckt die Stützmembran 101 vollständig. Die zweite Ausdehnung 1022 ist daher länger als die die zweite Ausdehnung 1012.

Die Oberflächen der Dichtmembranen 101, 102 können teilweise oder vollständig reibungsmindernd strukturiert oder beschichtet sein. Eine reibungsmindernde Ausführung im Bereich der Dichtmembranen 101, 102 kann eine Haft- und/oder Gleitreibung zwischen den Dichtmembranen 101, 102 reduzieren.

Eine reibungsmindernde Beschichtung an der inneren 101 und an der zweiten Dichtmembran 102 anliegenden Oberfläche der ersten Stützmembran 101 sowie an der äußeren und an der ersten Stützmembran 101 anliegenden Oberfläche der zweiten Dichtmembran 102 kann insbesondere die Haftreibung zwischen den Dichtmembranen 101, 102 reduzieren und damit Verschleiß verringern und die Dichtfunktion fördern.

### Fig. 10

In der Basis 104 entspringen die Dichtmembranen 101, 102, die beabstandet voneinander nach unten weisen und in Dichtlippen 111, 112 enden. Dabei ist die Dichtmembran 102, im Wesentlichen ohne Versatz, als Verlängerung der Wand 107 ausgeführt. Die Wandstärke der Dichtmembran 102 ist jedoch geringer als die Wandstärke der Wand 107 ausgeführt. Die Wandstärke der Dichtmembran 102 weist im weiteren Verlauf hin zu der Dichtlippe 112 eine gleichbleibende oder nur wenig abnehmende Wandstärke auf.

Dabei ist die Dichtmembran 101, im Wesentlichen mit einem Versatz, als Verlängerung der Wand 106 ausgeführt. Die Wandstärke der Stützmembran 101 entspricht im Wesentlichen der Wandstärke der Wand 108. Die Wandstärke der Stützmembran 101 weist im weiteren Verlauf hin zu der Dichtlippe 111 eine abnehmende Wandstärke auf.

Der Abstand zwischen Dichtmembran 102 und 101 ist über den Bereich der parallelen Erstreckung im Wesentlichen gleichbleiben bei 2 mm.

Der Abstand zwischen den Wänden 107 und 108 ist über den Bereich der parallelen Erstreckung im Wesentlichen gleichbleiben bei 2 mm.

### Fig. 11

Die Dichteinrichtung 100 weist ein näherungsweise U-förmiges Randprofil 103 auf, welches einen Befestigungsbereich zur formschlüssigen Verbindung der Dichteinrichtung 100 mit einem Maskenkörper 2 bildet. Das Randprofil 103 wird durch eine umlaufende, offene Nut 105 gebildet. Das Randprofil 103 weist zwei Profilwände 108, 107 auf die die Nut 105 bilden. Die Nut 105 ist trichterförmig 1051 gestaltet. Die Nut 105 ist dadurch trichterförmig 1051 gestaltet, dass zumindest eine der Profilwände 108, 107 einen abgeschrägten Wandabschnitt 1071 aufweist, der mit seiner Schräge (abgeschrägter Wandabschnitt) 1071 von der gegenüberliegenden Profilwand 108 zurückspringt.

Die Profilwand 107 bildet zudem eine Nase 106 aus. Die Nase 106 wird durch eine abschnittsweise Verdickung des Querschnitts der Profilwand 107 ausgebildet. Die Profilwand 107 weist einen abgeschrägten Wandabschnitt 1061 auf, der zu der Basis 1031 des U-förmigen Randprofils 103 weist. Der abgeschrägte Wandabschnitt 1061 ist in seiner Steigung relativ zur Wand 107 steiler ausgeführt als der Wandabschnitt 1071. Dadurch bildet der abgeschrägte Wandabschnitt 1061 einen Hinterschnitt aus für die Aufweitung am Gegenprofil 201 des Maskenkörpers 2. Zwischen der Nase und der Basis 1031 weist das Randprofil einen Aufnahmeraum 1032 für eine korrespondierende Verdickung 203 des Gegenprofils 201 auf.

Das Randprofil 103 kann ein (nicht gezeigtes) Gegenprofil 300 des Maskenkörpers aufnehmen. Damit bilden das Randprofil 103 und Gegenprofil 200 des Maskenkörpers eine formschlüssige Verbindung. Aufgrund der Elastizität des Randprofils 103 kann die Verbindung wiederholt hergestellt und gelöst werden. Zum Lösen der formschlüssigen Verbindung, insbesondere zum Herausziehen des Randprofils aus dem Gegenprofil 200 des Maskenkörpers, kann am Randprofil 103 oder an der Dichteinrichtung eine Lasche oder ein anderes Mittel vorgesehen sein, das beispielsweise mit zwei Fingern ergriffen werden kann, um das Randprofil 103 aus dem Gegenprofil 200 des Maskenkörpers zu ziehen.

Eine Nase 106 an einer Wand 107 des Randprofils 103 und eine korrespondierende Ausnehmung am Gegenprofil 200 des Maskenkörpers legen eine vorbestimmte relative Orientierung zwischen Dichteinrichtung 100 und Maskenkörper 2 fest. Die Wand 107 des Randprofils 103, die die Nase 106 aufweist ist kürzer als die zweite Wand 108.

Beide Wände 107 und 108 entspringen der Basis 104 des Randprofils 104 und weisen beabstandet voneinander nach oben und bilden so die Nut 105 zwischen sich.

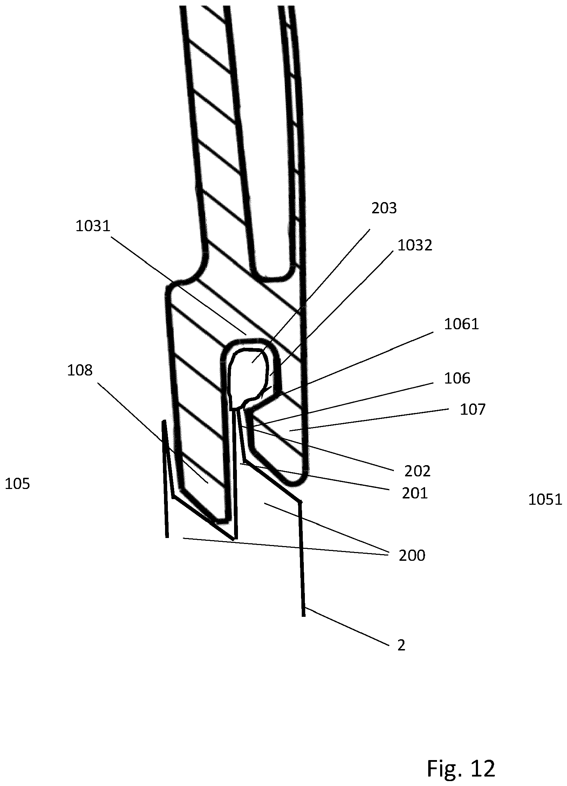

Fig. 12 zeigt die Dichteinrichtung 100 mit dem U-förmigen Randprofil 103 gemäß Fig. 12. zudem ist hier die formschlüssige Verbindung der Dichteinrichtung 100 mit einem Maskenkörper 2 dargestellt. Der Maskenkörper 2 weist dazu ein Profil 200 auf, welches so ausgebildet ist, dass es lösbar mit der Dichteinrichtung 100 verbunden werden kann und eine formschlüssige Verbindung mit der Dichteinrichtung 100 eingeht. Insbesondere weist das Profil 200 zumindest ein Gegenprofil 201 auf, welches in die Nut 105 eingeführt werden kann. Das Gegenprofil weist dafür einen Steg 202 auf an dessen Ende eine Verdickung 203 vorgesehen ist.

Die Verdickung 203 des Gegenprofils 201 ist so ausgebildet, dass sie in die Nut eingeführt werden kann. Die Verdickung 203 des Gegenprofils 201 kann in der Nut formschlüssig oder kraftschlüssig oder reibeschlüssig gehalten werden.

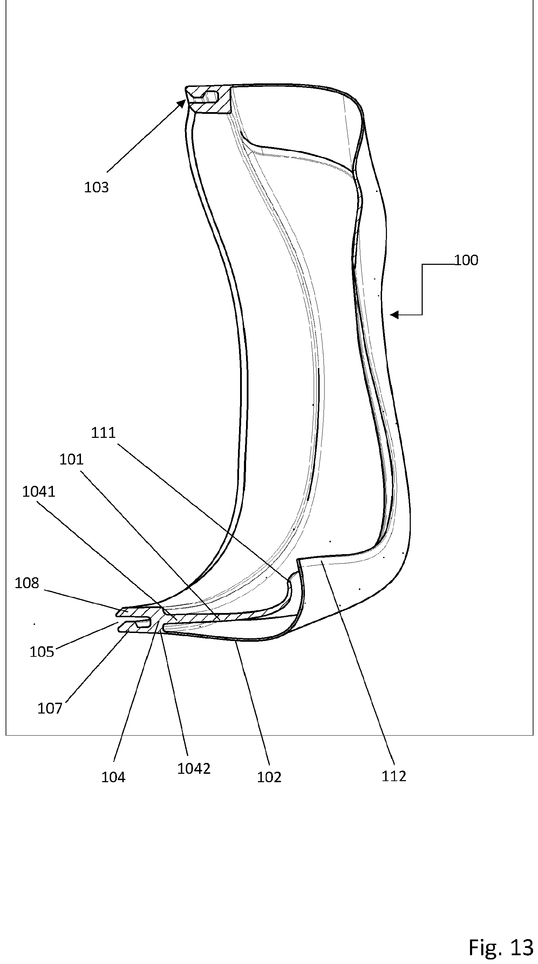

Figur 13 zeigt eine schematische Darstellung eines Schnitts durch eine Dichteinrichtung 100 gemäß Figur 3 an der Position 10. Der Schnitt geht daher durch die Vertikale der Dichteinrichtung 100 in y-Richtung.

Beide Wände 107 und 108 entspringen der Basis 104 des Randprofils 103 und weisen beabstandet voneinander in eine z-Richtung und bilden so die Nut 105 zwischen sich.

In der Basis 104 entspringen die Dichtmembranen 101, 102, die beabstandet voneinander in eine andere z-Richtung (entgegengesetzt zu 107, 108) weisen und in Dichtlippen 111, 112 enden. Dabei ist die Dichtmembran 102, im Wesentlichen ohne Versatz, als Verlängerung der Wand 107 ausgeführt. Die Dichtmembranen 101, 102 bilden einen elastischen mantelförmigen Wandabschnitt aus.

Die Wandstärke der Dichtmembran 102 ist jedoch geringer als die Wandstärke der Wand 107 ausgeführt. Die Wandstärke der Dichtmembran 102 weist im weiteren Verlauf hin zu der Dichtlippe 112 eine gleichbleibende oder nur wenig abnehmende Wandstärke auf. Die Wandstärke der Dichtmembran 102 liegt im Bereich 0,2 mm bis 0,5 mm, bevorzugt im Bereich 0,25 bis 0,45 mm, besonders bevorzugt im Bereich 0,3 mm bis 0,4 mm.

Die Wandstärke der Stützmembran 101 liegt an der Stelle 121, wo die Stützmembran 101 der Basis 104 entspringt, im Bereich 2,5 mm bis 1,4 mm, bevorzugt im Bereich 2,1 bis 1,6 mm, besonders bevorzugt im Bereich 2,0 mm bis 1,7 mm, ganz besonders bevorzugt bei 1,8 mm.

Die Wandstärke der Dichtlippen 111, 112 ist im Wesentlichen identisch.

Die Wandstärke der Stützmembran 101 liegt im Bereich der Dichtlippe 111 im Bereich zwischen 0,7 mm bis 0,35 mm, bevorzugt im Bereich 0,65 bis 0,4 mm, besonders bevorzugt im Bereich 0,6 mm bis 0,45 mm, ganz besonders bevorzugt bei 0,5 mm.

Der Abstand zwischen den Wänden 107 und 108 ist über den Bereich der parallelen Erstreckung im Wesentlichen gleichbleiben bei zumindest 1 bis 4 mm, bevorzugt bei 1,4 bis 3 mm, besonders bevorzugt bei 1,5 bis 1,8 mm.

Dabei ist die Dichtmembran 101, im Wesentlichen mit einem Versatz, als Verlängerung der Wand 108 ausgeführt. Die Wandstärke der Stützmembran 101 entspricht im Wesentlichen der Wandstärke der Wand 108. Die Wandstärke der Stützmembran 101 weist im weiteren Verlauf hin zu der Dichtlippe 111 eine abnehmende Wandstärke auf.

Der Abstand zwischen Dichtmembran 102 und 101 ist an der Basis bei 2 mm. Von dort aus verlaufen die Dichtmembranen 102 und 101 mit einem Winkel 125 von größer als 1°, bevorzugt von 1,3 bis 4°, besonders bevorzugt von 3° auseinander.

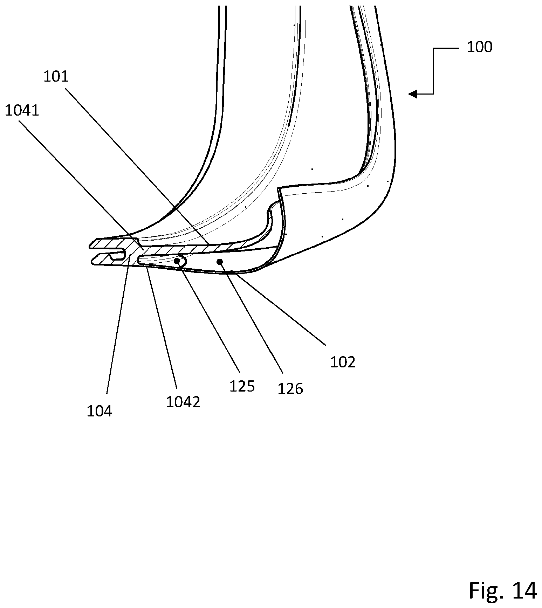

Figur 14 zeigt eine schematische Darstellung eines weiteren Ausführungsbeispiels einer Dichteinrichtung 100. Die Dichteinrichtung 100 ist in Figur 15 entlang einer Ebene aufgeschnitten dargestellt, die quer zur Längsachse vv der Dichteinrichtung 100 verläuft.

Die Dichteinrichtung 100 ist aus Silikon oder einem anderen elastischen Material hergestellt. Die Dichteinrichtung 100 umfasst eine erste Stützmembran 101 und eine zweite Dichtmembran 102.

Die zweite Dichtmembran 102 bildet einen elastischen, mantelartigen Wandabschnitt, der die Dichteinrichtung 100 außen begrenzt und die erste Stützmembran 101 zumindest teilweise überdeckt oder überlappt. Die Elastizität des elastischen mantelförmigen Wandabschnitts ermöglicht eine Bewegung der Dichtmembranen 101, 102 relativ zu den durch das Randprofil 103 gebildeten Befestigungsbereich der Dichteinrichtung 100, insbesondere eine Verschiebung senkrecht zur Längsachse und/oder ein Kippen oder Schwenken um Achsen senkrecht zur Längsachse.

Der Abstand zwischen den Dichtmembranen 101 und 102 ist über den Bereich der gemeinsamen Erstreckung im Wesentlichen gleichbleiben bei zumindest 1 bis 4 mm, bevorzugt bei 1,4 bis 3 mm, besonders bevorzugt bei 1,5 bis 1,8 mm oder bei 2 mm. Im dargestellten Ausführungsbeispiel ist der Abstand zwischen den Dichtmembranen 101 und 102 an dem Ursprung 1041, 1042 etwa 1,5 mm. Von dort aus verlaufen die Dichtmembranen 101 und 102 mit einem Winkel 125 von größer als 1°, bevorzugt von 1,3 bis 4°, besonders bevorzugt von 3° auseinander. Der Abstand zwischen den Dichtmembranen 101 und 102 nimmt über den Bereich der gemeinsamen Erstreckung - bedingt durch den Winkel 125 - zu und ist zumindest abschnittsweise bei zumindest 0,5 bis 5 mm, bevorzugt bei 1,4 bis 3 mm, besonders bevorzugt bei 1,5 bis 2,0 mm. Der leichte Winkel 125 führt dazu, dass die Dichtmembranen 101 und 102 nicht aneinanderkleben oder zueinander laufen, sondern divergieren. Dadurch wird eine Durchströmung des von den Dichtmembranen 101, 102 zumindest teilweise umgebenen Zwischenraums 126 mit Atemgas ermöglicht. Die Durchströmung des von den Dichtmembranen 101, 102 zumindest teilweise umgebenen Zwischenraums 126 mit Atemgas hat zumindest zwei vorteilhafte Wirkungen. Erstens kann der anliegende Therapiedruck die Dichtmembranen 102 zumindest leicht nach außen aufblähen oder gegen das Gesicht des Patienten drücken. Zweitens wird durch die Durchströmung des Zwischenraums 126 eine Ansammlung kondensierender Feuchtigkeit aus der Atemluft oder dem Atemgas zumindest abgemildert oder teilweise verhindert.

Eine Bewegbarkeit, insbesondere eine Verschiebbarkeit der Dichtmembranen 101, 102 und der Dichtlippen 111, 112 relativ zueinander wird durch die Elastizität des elastischen mantelförmigen Wandabschnitts der Dichtmembranen 101, 102 und der Dichtlippen 111, 112 bewirkt.

Eine Ausrichtung der Dichteinrichtung 100 auf einem nicht gezeigten Gesicht wird durch eine näherungsweise kuppelförmige Wölbung der Lichtlippen 111,112 bewirkt, wobei die Wölbung umlaufend in Richtung der Gesichtsöffnung 400 (Fig. 2) ausgeführt ist. Die kuppelförmige Wölbung der Dichtmembranen 101, 102 ist auch bei einer Verschiebbarkeit der Dichteinrichtung 100 möglich.

Für alle Ausführungsbeispiele gilt;
Die Stützmembran 101 kann ein Material aufweisen, das deutlich steifer oder weniger elastisch ist als das Material oder die Materialien der Dichtmembran 102. In diesem Fall kann die Materialstärke der Membran 101 in einem Querschnitt eine reduzierte aufweisen.

Die erste Stützmembran 101 kann mit dem Randprofil 103 gefügt, insbesondere an einer Kontaktstelle geklebt, geschweißt oder auf andere Weise stoffschlüssig gefügt. Alternativ können die Stützmembran 101 und das Randprofil 103 während eines Spritzgussvorgangs oder eines anderen Gussvorgangs miteinander stoffschlüssig gefügt werden.

Alternativ sind die Stützmembran 101 und das Randprofil 103 aus dem gleichen Material und insbesondere gleichzeitig und von Anfang an einstückig hergestellt. Auch die zweite Dichtmembran 102 und das Randprofil 103 können aus dem gleichen Material und insbesondere gleichzeitig und von Anfang an einstückig hergestellt sein.

## Patentansprüche

1. Patientenschnittstelle 1 mit einer Dichteinrichtung 100, wobei die Dichteinrichtung 100 eine Stützmembran 101 und eine Dichtmembran 102 aufweist, wobei die Dichtmembran 102 zumindest abschnittsweise zur Anlage an der Haut eines Patienten ausgebildet ist **dadurch gekennzeichnet, dass** die Stützmembran 101 zumindest abschnittsweise benachbart zur Dichtmembran 102 verläuft und wobei der Abstand zwischen der Stützmembran 101 und der Dichtmembran 102 zumindest abschnittsweise derart ausgebildet ist, dass ein Zwischenraum 126 gebildet wird.

2. Patientenschnittstelle 1 nach Anspruch 1 **dadurch gekennzeichnet, dass** die Dichteinrichtung 100 ein Randprofil 103 mit einer Basis 104 aufweist, wobei sich die Dichtmembran 102 und die Stützmembran 101 von der gemeinsamen Basis 104 ausgehend und beabstandet zueinander erstrecken.

3. Patientenschnittstelle 1 nach Anspruch 1 oder 2 **dadurch gekennzeichnet, dass** die Dichteinrichtung 100 ein näherungsweise U-förmiges Randprofil 103 aufweist, welches einen Befestigungsbereich 105 zur formschlüssigen Verbindung der Dichteinrichtung 100 mit einem Maskenkörper 2 bildet.

4. Patientenschnittstelle 1 nach zumindest einem der vorhergehenden Ansprüche **dadurch gekennzeichnet, dass** der Befestigungsbereich 105 durch eine umlaufende, offene Nut 105 gebildet ist.

5. Patientenschnittstelle 1 nach zumindest einem der vorhergehenden Ansprüche **dadurch gekennzeichnet, dass** das Randprofil 103 zwei Profilwände 108, 107 aufweist die die Nut 105 bilden, wobei die Nut 105 trichterförmig 1051 gestaltet ist.

6. Patientenschnittstelle 1 nach zumindest einem der vorhergehenden Ansprüche **dadurch gekennzeichnet, dass** die Nut 105 dadurch trichterförmig 1051 gestaltet ist, dass zumindest eine der Profilwände 108, 107 einen abgeschrägten Wandabschnitt 1071 aufweist, der mit seiner Schräge (abgeschrägter Wandabschnitt) 1071 von der gegenüberliegenden Profilwand 108 zurückspringt und die Profilwand 107 eine Nase 106 aufweist.

7. Patientenschnittstelle 1 nach zumindest einem der vorhergehenden Ansprüche **dadurch gekennzeichnet, dass** die Profilwand 107 einen abgeschrägten Wandabschnitt 1061 aufweist, der zu der Basis 1031 des U-förmigen Randprofils 103 weist, wobei der abgeschrägte Wandabschnitt 1061 einen Hinterschnitt für die Aufweitung am Gegenprofil 201 des Maskenkörpers 2 aufweist, wobei das Randprofil 103 so ein Gegenprofil 300 eines Maskenkörpers aufnehmen kann.

8. Patientenschnittstelle 1 nach zumindest einem der vorhergehenden Ansprüche **dadurch gekennzeichnet, dass** die Membranen 101, 102 von dem Randprofil 103 ausgehen und in eine von der Nut 105 abgewandte Richtung weisen, wobei von dem Randprofil 103 überall umlaufend eine Dichtmembran 102 entspringt und von dem Randprofil 103 bereichsweise keine Stützmembran 101 entspringt, die Stützmembran 101 also nicht durchgängig ausgebildet ist.

9. Patientenschnittstelle 1 nach zumindest einem der vorhergehenden Ansprüche **dadurch gekennzeichnet, dass** die Stützmembran 101 kürzer ausgebildet ist als die Dichtmembran 102, wobei die Stützmembran 101 zumindest abschnittsweise von der Dichtmembran 102 überdeckt wird.

10. Patientenschnittstelle 1 nach zumindest einem der vorhergehenden Ansprüche **dadurch gekennzeichnet, dass** ein Kontaktbereich 132 zur Haut eines Patienten durch die Dichtmembran 102 gebildet wird, die im Kontaktbereich 132 als Dichtlippe 112 ausgeformt ist.

11. Patientenschnittstelle 1 nach zumindest einem der vorhergehenden Ansprüche **dadurch gekennzeichnet, dass** die Dichtmembran 102 sich von der Basis 104 des Randprofil 103 ausgehend strahlförmig erstreckt und dann gerundet in eine Richtung abknickt, wobei die Dichtmembran 102 zumindest abschnittsweise einen rechten Winkel zu sich selbst bildet.

12. Patientenschnittstelle 1 nach zumindest einem der vorhergehenden Ansprüche **dadurch gekennzeichnet, dass** die Membranen 101, 102, beabstandet voneinander in der Basis 104 entspringen, wobei die Dichtmembran 102 als Verlängerung der Wand 107 ausgeführt ist, wobei die Wandstärke der Dichtmembran 102 geringer ist als die Wandstärke der Wand 107 und wobei die Stützmembran 101 zumindest abschnittsweise im Querschnitt dicker als die Dichtmembran 102 ist.

13. Patientenschnittstelle 1 nach zumindest einem der vorhergehenden Ansprüche **dadurch gekennzeichnet, dass** die Wandstärke der Dichtmembran 102 im weiteren Verlauf eine gleichbleibende oder nur wenig abnehmende Wandstärke aufweist.

14. Patientenschnittstelle 1 nach zumindest einem der vorhergehenden Ansprüche **dadurch gekennzeichnet, dass** die Stützmembran 101, im Wesentlichen mit einem Versatz, als Verlängerung der Wand 106 ausgeführt ist, wobei die Wandstärke der Stützmembran 101 im Wesentlichen der Wandstärke der Wand 106 entspricht, wobei die Wandstärke der Stützmembran 101 im weiteren Verlauf (hin zu der Dichtlippe 111) eine abnehmende Wandstärke aufweist.

15. Patientenschnittstelle 1 nach zumindest einem der vorhergehenden Ansprüche **dadurch gekennzeichnet, dass** die Wandstärke der Dichtmembran 102 im Verlauf hin zu der Dichtlippe 112 eine gleichbleibende oder nur wenig abnehmende Wandstärke aufweist, wobei die Wandstärke der Dichtmembran 102 im Bereich 0,15 mm bis 0,6 mm ist.

16. Patientenschnittstelle 1 nach zumindest einem der vorhergehenden Ansprüche **dadurch gekennzeichnet, dass** die Wandstärke der Stützmembran 101 an der Stelle 121, wo die Stützmembran 101 der Basis 104 entspringt, im Bereich 2,9 mm bis 1,4 mm liegt.

17. bevorzugt im Bereich 2,1 bis 1,6 mm, besonders bevorzugt im Bereich 2,0 mm bis 1,7 mm, ganz besonders bevorzugt bei 1,8 mm und wobei die Wandstärke der Stützmembran 101 im Bereich der Dichtlippe 111 im Bereich zwischen 0,7 mm bis 0,25 mm liegt.

18. Patientenschnittstelle 1 nach zumindest einem der vorhergehenden Ansprüche **dadurch gekennzeichnet, dass** die Wandstärke der Dichtlippen 111, 112 im Wesentlichen identisch ist.

19. Patientenschnittstelle 1 nach zumindest einem der vorhergehenden Ansprüche **dadurch gekennzeichnet, dass** der Abstand zwischen Dichtmembran 102 und der Stützmembran 101 an der Basis 104 bei 0,5 bis 3,5 mm liegt, wobei die Membranen 102 und 101 von dort aus mit einem Winkel 125 von größer als 1°, bevorzugt von 1,3 bis 4°, besonders bevorzugt von 3° auseinander laufen.

20. Patientenschnittstelle 1 nach zumindest einem der vorhergehenden Ansprüche **dadurch gekennzeichnet, dass** der Abstand zwischen den Membranen 101 und 102 ist über den Bereich der gemeinsamen Erstreckung im Wesentlichen bei zumindest 1 bis 4 mm, bevorzugt bei 1,4 bis 3 mm, besonders bevorzugt bei 1,5 bis 1,8 mm oder bei 2 mm liegt.

21. Patientenschnittstelle 1 nach zumindest einem der vorhergehenden Ansprüche **dadurch gekennzeichnet, dass** die Dichteinrichtung 100 aus Silikon oder einem anderen elastischen Material hergestellt ist, wobei die Dichteinrichtung 100 eine Stützmembran 101 und eine Dichtmembran 102 umfasst, wobei die Dichtmembran 102 einen elastischen, mantelartigen Wandabschnitt bildet der die Dichteinrichtung 100 außen begrenzt und die Stützmembran 101 zumindest teilweise überdeckt oder überlappt, wobei die Elastizität des elastischen mantelartigen Wandabschnitts eine Bewegung der Membranen 101, 102 relativ zu dem durch das Randprofil 103 gebildeten Befestigungsbereich der Dichteinrichtung 100 ermöglicht, insbesondere eine Verschiebung senkrecht zur Längsachse und/oder ein Kippen oder Schwenken um Achsen senkrecht zur Längsachse.

22. Patientenschnittstelle 1 nach zumindest einem der vorhergehenden Ansprüche **dadurch gekennzeichnet, dass** der Maskenkörper 2 und die Dichteinrichtung 100 zusammen einen Innenraum oder Atemgasraum 400 bilden, in welchen das Gesicht des Anwenders/Patienten bei der Anwendung der Maske auf dem Gesicht zumindest teilweise hereinragt, wobei der Innenraum der Patientenschnittstelle 1 über ein Ausatemsystem 3 mit dem Außenbereich der Patientenschnittstelle 1 gasleitend verbunden ist und wobei über einen Schlauchanschluss 5 die Patientenschnittstelle 1 über einen Beatmungsschlauch mit einem Beatmungsgerät gasleitend verbunden ist, wobei die Dichteinrichtung 100 einen Kontaktbereich 132 aufweist, der zur dichtenden Anlage an das Gesicht eines Patienten ausgebildet ist, der im Wesentlichen durch die Dichtlippe 102 gebildet ist.

23. Patientenschnittstelle 1 nach zumindest einem der vorhergehenden Ansprüche **dadurch gekennzeichnet, dass** der Kontaktbereich 132 einen Profilverlauf aufweist, der so ausgebildet ist, dass der Kontaktbereich 132 zur Anlage auf einem Nasenrücken und zur Anlage entlang einer Nasolabialfalte und entlang einer Oberlippe oder Unterlippe oder eines Kinnbereiches eines Patienten ausgebildet ist.
